# EUROPEAN PATENT APPLICATION

(11) **EP 4 653 544 A1**
(43) Date of publication of application: **26.11.2025**
(21) Application number: 24223671.9
(22) Date of filing: 30.12.2024
(51) Int. Cl.: C12Q 1/6818, C12Q 1/6804

(54) **A LABEL, MARKER AND METHOD FOR ANALYSING A BIOLOGICAL SAMPLE**

(30) Priority: 21.05.2024 EP 24177155
(71) Applicant: Leica Microsystems CMS GmbH, 35578 Wetzlar (DE); ATTO-TEC Gmbh, 57074 Siegen (DE)
(72) Inventor: Alsheimer, Soeren, 35578 Wetzlar (DE); Zilles, Alexander, 57074 Siegen (DE)
(74) Representative: Schaumburg und Partner Patentanwälte mbB

(57) **Abstract**

A label for analysing a biological sample is provided. The label comprises a first label part comprising a first nucleic acid strand (106) and a second label part comprising a second nucleic acid strand (110). The first nucleic acid strand (106) and the second nucleic acid strand (110) are configured to form a duplex. The label further comprises at least one first labelling moiety (108) and at least one second labelling moiety (112), and the label further comprises at least one blocking nucleic acid strand (126, 128). In further aspects, a marker (100, 500, 600) comprising the label and a respective method are provided.

## Description

### Technical field

The invention relates to a label and a corresponding marker. In a further aspect, a method for analysing a biological sample is provided.

### Background

Labels and markers are frequency used when analysing biological samples, for example in fluorescent microscopy. Markers for fluorescent microscopy generally comprise affinity reagents, such as antibodies, and labels attached to the affinity reagents, in order to enable specifically attaching the labels- in particular via the affinity reagents - to a target analyte in a biological sample. This allows the identification and/or localisation of the target analyte in the biological sample.

Detection in affinity reagent-based assays is usually performed by marking analytes with markers that generally comprise at least one affinity reagent and a (detectable) label, which may for example be an enzyme, a metal-tag, a Raman label, a fluorescent label or a hybrid label. A fluorescent label may comprise for example a fluorescent dye, a fluorophore, a fluorochrome, a dye, a QDot, a PDot, or a polymer dye. A detectable label may be, in particular, an optically detectable label.

Affinity reagents are used in life science to bind targets or analytes with high affinity and specificity. A variety of assays for liquid or solid samples rely on detection reagents that comprise an affinity reagent and a detectable label, which may comprise a nucleic acid, e.g. an oligonucleotide, a (fluorescent) dye, an enzyme, a metal tag, a radioactive tag, or an affinity tag (e.g. HA-, Myc-, FLAG-tags). Affinity reagents, such as aptamers, antibodies, and nanobodies, generally have dissociation constants (KD) in the pM to µM range and are used in a variety of affinity reagent-based assays or immunoassays, which are a pillar of life science research.

The various applications of markers include multiplexing approaches, which require large sets of distinguishable markers, whilst detecting analytes that only occur in small quantities requires markers with bright labels. In particular when handling large sets of markers, which may be assembled from individual components such as dyes and affinity reagents, it is of interest to reduce complexity of handling whilst maintaining flexibility of use and reliability.

### Summary

It is an object to provide a label and a marker that enables efficient handling when analysing biological samples.

The aforementioned object is achieved by the subject-matter of the independent claims. Advantageous embodiments are defined in the dependent claims and the following description.

In the sense of this document, a sample refers to a biological sample or specimen including for example blood, serum, plasma, tissue, bodily fluids (e.g. lymph, saliva, semen, interstitial fluid, cerebrospinal fluid), faeces, solid biopsy, liquid biopsy, explants, cells (e.g. prokaryotes, eukaryotes, archaea), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. In the sense of this document, sample may further refer to a volume surrounding a biological sample. For example, in assays, where secreted proteins like growth factors, extracellular matrix constituents are being studied, the extracellular environment surrounding a cell up to a certain assay-dependent distance is also referred to as a sample. Specifically, affinity reagents brought into this surrounding volume are referred to in the sense of this document as being introduced into the sample. Particularly relevant sample types for the present disclosure are formalin-fixed paraffin embedded (FFPE) tissue sections or tissue microarrays as well as tissue cryosections.

This allows the identification and/or localisation of the target analyte in the biological sample. An affinity reagent in the sense of this document may be for example an antibody, an antibody fragment, a nanobody, an aptamer, an aptabody, a polymeric binder, an affimer, an oligonucleotide probe (e.g. ISH or FISH probe), a drug/drug-like molecule or a toxin. An affinity reagent (AR) may also be a derivative of the above. An affinity reagent is usually configured to bind a target analyte or target with high specificity and affinity. However, many antibodies, which form the largest class of commercially available affinity reagents that bind proteins, are known to exhibit substantial cross-reactivity. In other words, many if not all antibodies do not only bind to their cognate target analyte, but also to a number of OFF-targets, i.e. targets that are not subject to the intended investigation. Both the cognate target against which an affinity reagent was produced or raised and the OFF-target may be collectively referred to as targets.

A further particularly relevant class of affinity reagents are aptamers and their derivatives. Aptamers are typically short nucleic acid sequences that fold into 3-dimensional structures capable of binding a target or analyte with high affinity and specificity. In the sense of this document, an aptamer refers to a small affinity reagent that is typically based on nucleic acids - either naturally occurring RNA, DNA or artificial ones XNAs, but may in some cases also be based on a peptide backbone. In the sense of this document, aptamers also refer to derivatives of aptamers such as SOMAmers, aptabodies, or other aptamer-derivatives, for example modified with modifications that are typically found on proteins such as glycosylation. Aptamers are generated by SELEX, can be produced at low cost with very high batch-to-batch consistency, practically unlimited shelf-life at -20°C, can be easily modified and are well suited to o a cyclic staining and imaging process described the European patent application with the application number 23178065.1, the complete content thereof being incorporated herein by reference. Both aptamers, antibodies, and nanobodies have dissociation constants (KD) in the pM to µM range and are used in a variety of affinity reagent-based assays or immunoassays, which are a pillar of life science research.

Generally, the label may be connected to the primary affinity reagent or a secondary affinity, which binds to the primary affinity reagent and thereby also introduces a certain degree of amplification. An affinity reagent may also be labelled with an oligonucleotide barcode, which can be addressed using a complementary oligonucleotide sequence that may be connected to a label. Using oligonucleotide barcodes and linkers provides an easy and efficient way not only to connect the elements of the marker, but also to incorporate additional functional elements like priming sites that can be used for amplification of barcode sequences by enzymatic amplification (e.g. polymerase chain reaction, PCR; loop-mediated isothermal amplification, LAMP; rolling circle amplification, RCA) or by hybridization chain reaction (HCR). In addition, or alternatively, landing sites for adapters may be incorporated that allow the formation of dendrimeric structures. This allows amplification of the signal, which is especially desirable when low abundance targets like PD1 or PD-L1 shall be analysed in tissue sections that have high background autofluorescence. In the sense of the present disclosure, which provides a novel way for paired detection the label comprises at least a first and second label part, which are connected directly or indirectly to a first and second affinity reagent.

A further challenge with using such markers is the cross-reactivity that affinity reagents display, i.e. they generally do not only bind to their target analyte also referred to as target or cognate target in the following, but may also recognize albeit typically with lower affinity other so called OFF-targets. This is the case for antibodies and other protein-binding affinity reagents as well as for oligonucleotide probes, which are configured to bind to nucleic acid targets.

The labels, markers, and methods described herein allow detecting the proximity of multiple analytes in a biological sample. Said analytes may be part of the same protein in which case the method may be used to study post-translational modifications such as phosphorylation, ubiquitination, and acetylation for example or said analytes may be different proteins in which case the method may be used to determine their proximity in the sample as a proxy for protein-protein interaction. Further, said method may be used to probe multiple epitopes on the same target protein to enhance the specificity of the detection, or may be used to detect the presence of multiple epitopes on the same pathogen e.g. E. coli or virus to determine the respective strain with very high specificity. In other words, the present disclosure provides a proximity assay, which may be referred to as **Proximity Hybridization Assay** (PHA).

Earlier, a solution of this problem was provided that is based on collecting cross-reactivity profiles and computational cross-reactivity unmixing as described in the European patent application with the application number 23190568.8, the complete content thereof being incorporated herein by reference. In the present document, an assay format is proposed that is particularly suitable to detecting the binding of pairs of affinity reagents to a target analyte. This method may be used to determine for example a high number of protein-protein-interactions in tissue samples, which may be referred to as "spatial interactomics".

As fields like cytometry, plasma proteomics, and microscopy are evolving towards higher levels of plexity, the problem of cross reactivity has come more into focus and has been recognized by the scientific community as a key component of the so-called reproducibility crisis. In a position paper called "Reproducibility: Standardize antibodies used in research"published by Bradbury and Pluckthin in Nature 518, 27-29 (2015) with 110 cosignatories, it was stated that fewer than half of around 6,000 routinely used commercial antibodies recognized only their specified targets. More recent work by Schwenk et al. (Toward Next Generation Plasma Profiling via Heat-induced Epitope Retrieval and Array-based Assays, Molecular & Cellular Proteomics, Volume 9, Issue 11, 2497 - 2507) analysed 11,000 affinity-purified, monoclonal antibodies and found only 531 to produce a single band on a western blot. While a systematic and comprehensive analysis of the phenomenon has not been performed, the advent of antibody microarrays has generated new possibilities to probe crossreactivity of affinity reagents in a practical assay format. From a conceptual point of view, it is therefore very well possible that we will never be able to make a genuinely specific affinity reagent, i.e. an affinity reagent that truly binds to only one target. Bradbury, A., Plückthun, A. (Reproducibility: Standardize antibodies used in research, Nature 518, 27-29, (2015), https://doi.org/10.1038/518027a) further state that cosignatories were able to replicate the scientific results of only 6 of 53 landmark preclinical studies and went on to predict that this phenomenon is costing an estimated $350 million per year in the United States alone.

### Paired detection

Paired detection, wherein the same analyte or multiple analytes may be detected, may be used in biochemical assays for various means. Typically, antibody pairs are used in conjunction with an assay like for example the proximity extension assay (PEA) from OLINK (Uppsala, Sweden) to detect the presence of an analyte in a liquid like serum with high specificity or proximity ligation assay (PLA) from Navinci (Uppsala, Sweden) to detect proximities of two distinct proteins. Matched antibody pairs for paired detection assays are readily available from suppliers like abcam, which presently supplies more than 1,800 antibody pairs.

The present method disclosed herein may be referred to as **Proximity Hybridisation Assay (PHA)** and is an assay designed to allow faithful detection of analyte proximities in biological samples to either
(A) **increase the specificity** of the assay by mitigating false-positive events arising from cross-reactivity or cross-hybridization
(B) interrogate
   - post-translational modifications,
   - alternative splicing, or
   - proteolytic processing.

Proximity Hybridization Assay (PHA) therefore is relevant both in terms of detecting proximities of two distinct molecules (e.g. two different proteins) as well as might be used to detect the same protein using paired-detection for example with two antibodies that recognize two distinct epitopes on the same protein or two sequence stretches on the same RNA or DNA locus.

### Blocking strand controlled hybridization as a strategy to inhibit duplex formation during target binding and removing of unbound affinity reagents and label parts

An important aspect of the present disclosure can be regarded in that **blocking strand controlled hybridization** can be leveraged **to render the hybridization or duplex formation of the complementary first and second label parts controllable.** This is necessary to avoid label part-mediated aggregation of the affinity reagents e.g. antibodies, in absence of the targets, which would otherwise disturb the assay, and enables forming the duplex only after the unbound marker and label parts have been washed out. In this way, a duplex can only form, when a pair of affinity reagents, e.g. antibodies, is bound to two targets that are in close proximity, wherein close proximity is defined as being in a radius of around up to 1-10, 5-20, 20-100, or 100-1,000nm radius depending on the configuration of the assay. For example, for a co-detection of two surface markers on a bacterium the spatial stringency may be chosen to be lower for example 100-1,000nm. While for an assay for post-translational modifications nanobodies, short linkers, and shorter first and second label parts may be used to bring the spatial stringency, i.e. required analyte proximity to generate a positive test result, into the range of 5-20nm. For the highest spatial stringency, one or both affinity reagent may be a drug, toxin, or other small molecule (e.g. hormone, neurotransmitter) known to bind to the target with high affinity and specificity. In the sense of this document high affinity refers to KD in the range of µM to fM. In the sense of this document an affinity reagent is regarded specific, when the skilled biologist, biochemist, or molecular biologist would regard the affinity reagent as specific. As proteome-wide systematic binding studies generally have not been performed for antibodies or other affinity reagents, it is clear that a specific affinity reagent in the sense of this document may well have OFF-targets that it binds to although typically less strong than to its cognate target.

### Detection of the duplex formation and thereby label formation

The label of the present disclosure, which may also be referred to as an PHA-label, comprises a first label part and a second label part. These label parts further comprise a first nucleic acid backbone also named first nucleic acid strand and a second nucleic acid backbone also named a second nucleic acid strand, for example oligonucleotide backbones configured to hybridize with each other. To inhibit the formation of the duplex between the first and second label part either one of them or both of them are hybridized to a so called first and/or second blocking strand. This blocking strand is configured to block the duplex formation between the first and second label part during target binding and washing out of unbound marker parts. The blocking strand is then removed in a subsequent step to allow formation of the label due to the formation of the duplex between the at least one first and second nucleic acid strands. The removal of the blocking strand may be brought about in different ways: (1) enzymatic digest using for example restriction enzymes, exonucleases like DNase I, RNase H or similar, (2) melting off, (3) facilitated melting by modification of at least one of the first or second blocking strands or the first or second nucleic acid strands with a guest-molecule configured to mediate duplex invasion by complexation with a suitable host molecule, (4) strand displacement, (5) chemical cleavage for example using disulfide-bond modified blocking strands and a reducing agent like TCEP, (6) blocking strands that comprise UV-cleavage sites and UV light exposure.

In a first aspect, a label for analysing a biological sample is provided. The label comprises a first label part comprising a first nucleic acid strand, preferably a single stranded nucleic acid, and a second label part comprising a second nucleic acid strand, preferably a single stranded nucleic acid. The first nucleic acid strand and the second nucleic acid strand are configured to form a duplex, in particular, the first nucleic acid strand and the second nucleic acid strand are at least partially complementary to each other. Thus, the first nucleic acid strand and the second nucleic acid strand may hybridise and form the duplex or double stranded structure, for example. In particular, the first nucleic acid strand and the second nucleic acid strand may hybridise to each other along their entire length. The label further comprises at least one first labelling moiety and at least one second labelling moiety, and the label further comprises at least one blocking nucleic acid strand, preferably a single strand nucleic acid, which is at least partially complementary to one of the first nucleic acid strand and the second nucleic acid strand. Thus, the blocking nucleic acid strand may form a duplex or double stranded structure with the respective first nucleic acid strand or second nucleic acid strand.

The label is preferably configured to be optically detectable at least when the first label part and second label part are in close proximity. The blocking nucleic acid strand enables undesired or uncontrolled hybridisation between the first and second label parts. In turn, this enables efficiently handing of the labels parts, for example, when preparing to analyse a biological sample. For example, when using the label to analyse the proximity between target analytes of a biological sample.

In particular, the labelling moieties are (covalently) attached to one of the first and second nucleic acid strands. For example, the first and second labelling moieties may be a fluorescence resonance energy transfer (FRET) pair, which is optically detectable when they are in close proximity, in particular within 1 to 10 nm. This may be used for FRET-based PHA assays, in which the FRET efficiency between different label parts of a marker may be measured by performing readouts in the presence and absence of blocking strand(s), i.e. before and after the removal of blocking strands. FRET may be measured by reading intensities and/or changes in fluorescence lifetime. Devices like the STELLARIS confocal microscope from Leica Microsystems (Mannheim, Germany), which is equipped with a white-light laser (WLL) pulsed light source and FALCON fluorescence lifetime imaging module to measure the change in fluorescence lifetime (FLIM-FRET; further detail below), are well suited for this task.

The first and second labelling moieties may be of the same or of different types and may therefore have the same or different optical properties, such as excitation wavelength, emission wavelength and emission lifetime. The at least one first labelling moiety and at least one second labelling moiety may be the same, i.e. the same fluorescent dye such as ATTO647N, for example. The at least one first labelling moiety and at least one second labelling moiety may each be connected covalently to either the first or second nucleic acid strand or may each be connected to one of the first or second nucleic acid strand. Preferably, the at least one first labelling moiety and/or the at least one second labelling moiety preferably comprise identical fluorescent dyes. In particular, the fluorescent characteristics of the at least one first labelling moiety and/or the at least one second labelling moiety is essentially the same. Alternatively, the at least one first labelling moiety comprise at least one first fluorescent dye and the at least one second labelling moiety comprise at least one second fluorescent dye, the at least first and second fluorescent dye having different characteristics. The characteristic may be different regarding the excitation, emission and/or fluorescent lifetime of the at least first and second fluorescent dye.

Preferably, the at least one first labelling moiety and/or the at least one second labelling moiety is optically detectable. This enables efficient detection of the label, for example, by means of a microscope, in particular a fluorescence microscope. For example, the first labelling moiety and/or the second labelling moiety may comprise a fluorophore such as fluorescent proteins, organic or inorganic fluorescent molecules, or fluorescent nanoparticles.

Preferably, the at least one blocking nucleic acid strand is, in particular selectively, degradable by means of a degradation agent and wherein the first nucleic acid strand and the second nucleic acid strand are resistant to the degradation agent. This enables efficiently removing the blocking nucleic acid strand by addition of the degradation agent. In particular, when the blocking nucleic acid strand is hybridised to the respective first or second nucleic acid strand, the degradation agent enables removing the blocking nucleic acid strand to enable the hybridisation of the first and second nucleic acid strands.

Preferably, the at least one blocking nucleic acid strand consists (essentially) of one of a nucleic acid analogue or a natural nucleic acid, and the first nucleic acid strand and the second nucleic acid strand consist (essentially) of the other one of a nucleic acid analogue or a natural nucleic acid. This enables selectively degrading the blocking nucleic acid strand or the first and second nucleic acid strands, for example, in an iterative staining process or to selectively remove the blocking nucleic acid strand.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid. Whereas natural nucleic acids or naturally occurring nucleic acids (DNA and RNA) are generally sensitive to degradation agents such as nucleases, nucleic acid analogues are generally resistant to degradation agents such as nucleases. Additional examples of nucleic acid analogues are PT-DNA, morpholino, PNA, and L-DNA.

In a particular embodiment, at least one of the blocking nucleic acid strand, the first nucleic acid strand, and the second nucleic acid strand comprises a nucleic acid analogue. For example, the blocking nucleic acid strand may comprise modified/analogous nucleotides comprising disulfide bridges that are configured to function as TCEP (tris(2-carboxyethyl)phosphine) cleavage sites and that can be cleaved by the application of TCEP resulting in the degradation of the blocking nucleic acid strand and its removal from the first nucleic acid strand or the second nucleic acid strand. Alternatively or additionally, the blocking nucleic acid strand may comprise UV-cleavable modified/analogous nucleotides or wherein the blocking nucleic acid strand comprises H₂O₂-cleavable modified nucleotides, for example arylboronic acid ester modifications.

Preferably, the at least one blocking nucleic acid strand or the entire blocking nucleic acid strand or a part thereof is complementary to at least a part of the first nucleic acid strand or the second nucleic acid strand. Thus, the blocking nucleic acid strand might be entirely complementary to the respective part of the first or second nucleic acid strand. In particular, all nucleotides of the blocking nucleic acid strand may hybridise to complementary respective nucleotides of the first or second nucleic acid strand. This enables efficiently blocking a hybridisation between the first and second nucleic acid strands when the blocking nucleic acid strands is present. In particular, the blocking nucleic acid strand hybridises to all nucleotides of the first or second nucleic acid strand.

Preferably, the at least one blocking nucleic acid strand is complementary to the part of the first nucleic acid strand or the part of the second nucleic acid strand that are complementary to each other, in particular complementary to the respective parts that form the duplex with each other. This enables efficiently blocking a hybridisation between the first and second nucleic acid strands when the blocking nucleic acid strands is present.

Preferably, the label comprises a plurality of blocking nucleic acid strands that each bind to a different part of the first nucleic acid strand or to a different part of the second nucleic acid strand. In particular, the blocking nucleic acid strands may each comprise between 5 and 15 nucleotides.

Preferably, the at least one first labelling moiety and the at least one second labelling moiety are each configured for non-radiative energy transfer between them, in particular when they are in close proximity. This enables efficiently determining whether the label parts are in close proximity to each other. For example, the first labelling moiety and the second labelling moiety may be configured to form a FRET pair with each other, where one is the FRET donor and the other is the FRET acceptor. The FRET between the first labelling moiety and the second labelling moiety may generally occur when the label parts are hybridised to each other and therefore the labelling moieties are in close proximity.

Preferably, the at least one first labelling moiety and the at least one second labelling moiety are both (covalently) attached to either the first nucleic acid strand or the second nucleic acid strand. For example, they may be attached to the phosphate backbone of the respective nucleic acid. In particular, the labelling moiety may be attached to opposite ends of the respective first or second nucleic acid strand. For example, the at least one first labelling moiety may be attached towards a 3' end of the respective nucleic acid strand and the second labelling moiety may be attached towards a 5' end of the respective nucleic acid strand.

In the above case, the first and second labelling moieties are preferably hydrophobic labelling moieties. Generally, in an aqueous solution hydrophobic labelling moieties have a tendency to aggregate. In particular, hydrophobic labelling moieties that are arranged in close proximity on a flexible single stranded nucleic acid strand may lead to formation of aggregates. This aggregation may lead to self-quenching or changes to the optical properties of these labelling moieties such as their absorption wavelength and/or in particular their fluorescence brightness, compared to non-aggregated hydrophobic labelling moieties. The formation of the duplex between the first and second nucleic acid strand when they are in close proximity results in an increase in the degree of rigidity or stiffness of the (first and second) nucleic acid strand. A measure of the degree of rigidity or stiffness of a nucleic acid strand, in particular of a duplex structure, is persistence length (Lₚ). The persistence length is a mechanical parameter quantifying polymer rigidity: the higher the persistence length, the more rigid the polymer. In the presence of monovalent or divalent salts, a nucleic acid duplex structure regularly has a persistence length of 30 to 55 nm, while a single stranded nucleic acid is much more flexible, with a persistence length of 1.5 to 3 nm. Thus, a double stranded nucleic acid has a higher persistence length and correspondingly a more linear form.

In particular, when the first labelling moiety and the second labelling moiety are attached on opposing ends of the first or second nucleic acid strand, the increase in rigidity of the respective nucleic acid strand may result in an increase in the distance between the first and second labelling moiety. Thus, previously aggregated labelling moieties are separated by hybridisation of the first and second nucleic acid strands. This separation may result in a detectable change in the optical properties of the first labelling moiety and the second labelling moiety. Consequently, the hybridisation between the first and second nucleic acid strands, due to the proximity of the first and second label parts, is detectable by the change in the optical properties of the labelling moieties.

Examples of hydrophobic labelling moieties that are regularly used to analyse biological samples include ATTO 390, ATTO 425, ATTO 550, ATTO Rho12, ATTO 633, and ATTO 647N.

Aside from FRET the PHA assay described in this disclosure may also be based on dequenching. A PHA assay may be setup with a first marker part comprising a first label part that comprises both the at least one first and at least one second labelling moiety. For example, the first label part may comprise a plurality of ATTO674N dye molecules. In this case the first label part will be strongly self-quenched due to the tendency of the hydrophobic dye molecules to aggregate and the flexibility of the first nucleic acid strand. This is described in the European patent application with the application number EP24177155.9, the complete content thereof is herein incorporated by reference. In a dequenching-based PHA assay the quenched label part for example the first label part is dequenched by the duplex formation with the second label part, which necessitates the proximity of the first and second label part. Therefore, the dequenching efficiency can be taken as a measure for the proximity of the first and second marker parts in this case.

Preferably, the at least one first labelling moiety is (covalently) attached to the first nucleic acid strand, for example to a phosphate backbone of the nucleic acid strand, and the at least one second labelling moiety may be (covalently) attached to the second nucleic acid strand, for example to a phosphate backbone of the nucleic acid strand. In this case, the optical properties of the first and second labelling moieties may similarly change due to their proximity when the first and second nucleic acid strands are hybridised to each other.

Preferably, the first nucleic acid strand extends along a first direction and a plurality of the first labelling moieties are arranged on the first nucleic acid strand along the first direction, and/or the second nucleic acid strand extends along a second direction and a plurality of the second labelling moieties are arranged on the second nucleic acid strand along the second direction. This enables generating an optically detectable signal that is proportional to the distance between the first label part and the second label part. In particular, the respective labelling moieties are arranged one after another along the respective direction. In particular, the respective labelling moieties are arranged one after another.

Preferably, each labelling moiety is essentially equally spaced from any adjacent labelling moiety. This enables generating an optically detectable signal that is proportional to the distance between the first label part and the second label part. When the label comprises a plurality of labelling moieties, each first labelling moiety is substantially equally spaced from any adjacent first labelling moiety, and/or each second labelling moiety is substantially equally spaced from any adjacent second labelling moiety. Preferably, the essentially equal spacing may be in a range from 0.33nm to 33 nm.

Preferably, the label further comprising at least one guest molecule configured to form a complex with a host molecule. In particular, the guest molecule may be (covalently) attached to the blocking nucleic acid strand. For example, to its phosphate backbone or a nucleobase. The guest-host complex formation may disrupt or hinder duplex formation of the respective nucleic acid strand the guest molecule is attached to. This enables control over the hybridisation of the respective nucleic acid strand.

The guest molecule is preferably covalently attached to one of the (first and second) nucleic acid strands. In particular, the label may comprise a plurality of guest molecules. In this case, one guest molecule may bind to one host molecule. In particular, the at least one guest molecule is not a nucleotide and/or not a labelling moiety. In a particular embodiment, there may be one guest molecule for every ten nucleotides of one of the nucleic acid strands.

The guest molecule is preferably configured to selectively form a complex with the host molecule. Thus, the guest molecule may be configured to form the complex with the host molecule only under a particular complexing condition. Similarly, the guest molecule may be configured to decomplex from the host molecule only under a decomplexing condition, which is different to the complexing condition. The complex formation may include binding of the guest molecule to the host molecule. For example, the complex formation may be based on intermolecular forces such as hydrogen bonding, Van der Waals forces, or dipole interactions. Preferably, the complex formed between the host molecule and the guest molecule is configured to disrupt or hinder hybridisation of the first nucleic acid strand and the second nucleic acid strand to each other or of the blocking nucleic acid strand to the respective first or second nucleic acid strand. In particular, the complex may reduce the melting temperature of the duplex of the hybridised nucleic acid strands.

Preferably, the at least one guest molecule is configured to form the complex with the host molecule under a complexing condition. Similarly, the guest molecule and/or the host molecule are configured to decomplex under a decomplexing condition. Such a decomplexing condition and/or complexing condition may include varying a concentration of the host molecule or addition of a competitor guest molecule, varying pH, salt, and/or temperature. This enables selectively forming the complex between the host molecule and the guest molecule. In particular, the complexation of the at least one guest molecule with the host molecule may lead to a strand invasion and a dissociation of the duplex, in particular between the blocking nucleic acid strand and the first or second nucleic acid strand.

Preferably, the label comprises a plurality of the guest molecules, wherein the guest molecules may be evenly spaced along the blocking nucleic acid strand. This enables efficient control over the hybridisation of the first nucleic acid strand or the second nucleic acid strand to the blocking nucleic acid strand by means of the host molecule. Preferably, one guest molecule is provided per every 5 to 10 nucleotides of the blocking nucleic acid strand.

Preferably, the at least one guest molecule is one of 1-adamantanemethylamine, ferrocenyl methylamine, 1,4-benzenedimethanamine, and 4-tertbutylbenzylamine. Xiao et al, 2022 (Controllable DNA hybridization by host-guest complexation-mediated ligand invasion. Nature Communications 13: 5936) provides further details of suitable host- and guest-molecules.

Preferably, the label comprises at least one host molecule. Providing the label with the at least one guest molecule and host molecule enables avoiding random or spontaneous hybridisation of the first nucleic acid strand or the second nucleic acid strand to the blocking nucleic acid strand. In particular, this enables controlling the hybridisation between the respective nucleic acid strands. This makes the introduction of the label parts into a biological sample easier and more efficient. In particular, the label may comprise a plurality of host molecules to at least form a complex with each guest molecule. The host molecule may be configured to form a complex with the guest molecule.

Preferably, the host molecule is a cucurbit[n]uril, in particular cucurbit[7]uril. In particular, the host molecule does not comprise nucleotides or nucleic acids.

In a further aspect, a marker for analysing a biological sample with a plurality of target analytes is provided. The marker comprises a label, in particular as described above, comprising a first label part and a second label part. A first marker part comprises a first affinity reagent and the first label part, and a second marker part comprising a second affinity reagent and the second label part. The first affinity reagent and the second affinity reagent are each configured to bind specifically to one of the target analytes of the biological sample.

The marker enables efficiently determining the distance or proximity between two target analytes. The marker also enables precisely determining the presence and/or location of the two target analytes or of a single target analyte in a biological sample. The biological sample may be a cellular sample, such as a tissue section, for example.

Further examples include biopsy samples such as liquid biopsy or a tissue biopsy. The target analyte may be a protein or a nucleic acid of the biological sample, for example.

The affinity reagents of the marker may be antibodies, antibody fragments, amino acid- or nucleic acid-based aptamers, or linear nucleic acids. This enables detecting a large variety of target analytes. In particular, the nucleic acid strand of a label part is attached to the respective affinity reagent, preferably covalently attached.

The affinity reagents of the marker may bind to a single target analyte or to separate target analytes. In the first case, the first affinity reagent may be configured to bind specifically to a first area or epitope of the single target analyte and the second affinity reagent may be configured to bind specifically to a second area or epitope of the single target analyte. By requiring two marker parts to bind to the single target analyte, the presence and/or location of the single target analyte may be precisely determined, in particular with an increased specificity. In the second case, the first affinity reagent may be configured to bind specifically to a first target analyte and the second affinity reagent may be configured to bind specifically to a second target analyte. This enables determining the distance or proximity between the first target analyte and the second target analyte.

In another aspect, a method for analysing a biological sample is provided. The method comprises the step of introducing at least one marker, in particular as described above, into the biological sample. In a next step, the blocking nucleic acid strand is removed from the marker. Finally, an optical readout of the biological sample with the marker is generated, for example by means of a microscope, such as a fluorescence microscope. The optical readout enables determining presence and/or location of a target analyte or a distance between target analytes.

The step of introducing the at least one marker may optionally include waiting for a set amount of time to allow binding of the at least one marker to a respective target analyte of the biological sample. After introducing the at least one marker, any marker not bound to the target analyte may optionally be removed from the biological sample, for example by washing. When introducing the at least one marker, the marker comprises the blocking nucleic acid strand, preferably the blocking nucleic acid strand is hybridised to the respective first or second nucleic acid strand of the label of the marker. The step of introducing the at least one marker may include adding individual parts of the at least one marker to the sample separately, in particular separately over time. After the step of introducing the at least one marker, preferably after the binding of the at least one marker to a respective target analyte of the biological sample and in particular after any marker not bound to the target analyte has been removed from the biological sample, for example by washing, an optical readout of the biological sample with the marker might be generated, in order to optically read out the single label parts (e.g. the first and the second label part) before the next method step - the removal of the blocking nucleic acid strand from the marker - is performed. While it may not always be required, it is generally advisable to perform at least two optical readouts of the biological sample: one wherein the first and/or second label part are still hybridized to the blocking nucleic acid strands which may also be named blocking strands and one after the removal of the blocking strands. In this way a fold change or ratio can be calculated.

The step of removing the blocking nucleic acid strand may include one of the following: applying a degradation agent configured to selectively degrade the blocking nucleic acid strand, and/or applying a host molecule, in particular under a complexing condition, to the biological sample, in particular in case the blocking nucleic acid strand comprises guest molecules.

Alternatively or in addition, the step of removing the blocking nucleic acid strand may include melting the blocking nucleic acid strand away from the respective first or second nucleic acid strand of the label of the marker, for example by increasing a temperature the sample and marker is at. In addition, the blocking nucleic acid strand may be removed from the sample by washing the sample.

Removal of the blocking nucleic acid strand enables the hybridisation, and therefore duplex formation, between the first and second nucleic acid strand of the label of the marker.

In particular, the at least one marker introduced into the biological sample includes the at least one guest molecule, in particular under a decomplexing condition. The decomplexing condition may include adjusting a salt concentration, adjusting temperature, and/or addition of a competitive guest molecule that is not attached to the marker, for example. This avoids generating the guest-host complex and allows hybridisation of the blocking nucleic acid strand to the first or second nucleic acid strands of the marker parts. However, this enables forming a complex with the guest molecule and a host molecule in subsequent steps of the method.

Preferably, during the step of removing the blocking nucleic acid strand, the host molecule may be introduced into the sample, in particular under a complexing condition. The complexing condition enables the formation of a complex between the guest molecule and the host molecule and thereby avoid hybridisation of or effect the dissociation of the blocking nucleic acid strand and the first or second nucleic acid strand of the marker.

Generally, a plurality of markers may be introduced into the biological sample, the markers being specific to respective target analytes or pairs of target analytes, in order to identify a large number of (different or the same) target analytes at the same time. Preferably, a target analyte is identified and/or localised within the biological sample based on the label(s) of the marker(s), in particular the labelling moieties, associated with the target analyte in the optical readout. Markers may be physically constituted before introducing them into the biological sample. Alternatively, affinity reagents may be barcoded with oligonucleotide barcodes and introduced into the biological sample initially and the labels comprising complementary barcode oligonucleotides may be introduced into the biological sample in a subsequent step and may then attach to their respective affinity reagent thereby forming the respective markers within the biological sample.

In another aspect, a kit for analysing a biological sample is provided. The kit comprises a marker, in particular as described above, and a degradation agent configured to selectively degrade the blocking nucleic acid strand of the marker or at least one host molecule configured to form a complex with the at least one guest molecule of the marker.

The label, the marker, and the kit may preferably be used for a proximity assay for analysing a biological sample.

The marker, kit and the method have the same advantages as the label. Further, the marker, kit and the method may be supplemented with the features of the label described in this document, in particular, the features of the dependent claims of the label. In particular, a use of a label in particular as described above, or a use of a marker in particular as described above, or a use of a kit in particular as described above for a proximity assay for analysing a biological sample, is possible.

### Terms and Definitions

In the sense of this document, the term **"affinity reagent"** refers to any reagents that is configured to bind a target in a biological sample with high affinity and specificity. In particular the term **"affinity reagent"** refers to antibodies, nanobodies, aptamers, affimers, Protein A, Protein G, streptavidin, oligonucleotide probes (e.g. FISH probes) molecularly imprinted polymers, nanoMIPs, smart polymers, DARPINs, or other forms of polymeric or artificial binders that are configured to recognize at target such as a protein, RNA, DNA, peptide, neurotransmitter, hormone, metabolite in a biological sample. An **"affinity reagent"** is typically used to label a target analyte in a biological sample to render it optical detectable or separable by means of purification.

In the sense of this document, the terms **"fluorescent dye", "fluorophore", "fluorochrome", "dye"** are used interchangeably to denote a fluorescent chemical compound or structure and can be in particular one of the following: a fluorescent organic dye, a fluorescent quantum dot, a fluorescent dyad, a fluorescent carbon dot, graphene quantum dot or other carbon-based fluorescent nanostructure, a fluorescent protein, a fluorescent DNA origami-based nanostructure. From the organic fluorescent dyes in particular derivatives of the following are meant by the term "fluorescent dye": xanthene (e.g. fluorescein, rhodamine, Oregon green, Texas), cyanine (e.g. cyanine, indocarbocyanine, oxacarbocyanine, thiacarbocyanine, merocyanine), derivatives, squaraine rotaxane derivatives, naphthalene, coumarin, oxadiazole, anthracene (anthraquinones, DRAQ5, DRAQ7, CyTRAK Orange), pyrene (cascade blue), oxazine (Nile red, Nile blue, cresyl violet, oxazine 170), acridine (proflavine, acridine orange, acridine yellow), arylmethine (auramine, crystal violet, malachite green), tetrapyrrole (porphin, phthalocyanine, bilirubin), dipyrromethene (BODIPY, aza-BODIPY), a phosphorescent dye, or a luminescent dye. The following trademark groups denote commercially available fluorescent dyes, which may include dyes belonging to different chemical families such as CF dye (Biotium), DRAQ and CyTRAK probes (BioStatus), BODIPY (Invitrogen), EverFluor (Setareh Biotech), Alexa Fluor (Invitrogen), Bella Fluore (Setareh Biotech), DyLight Fluor (Thermo Scientific), Atto and Tracy (Sigma-Aldrich), FluoProbes (Interchim), Abberior Dyes (Abberior Dyes), Dy and MegaStokes Dyes (Dyomics), Sulfo Cy dyes (Cyandye), HiLyte Fluor (AnaSpec), Seta, SeTau and Square Dyes (SETA BioMedicals), Quasar and Cal Fluor dyes (Biosearch Technologies), SureLight Dyes (Columbia Biosciences), Vio Dyes (Milteny Biotec). From the group of fluorescent proteins in particular the members of the green fluorescent protein (GFP) family including GFP and GFP-like proteins (e.g DsRed, TagRFP) and their (monomerized) derivatives (e.g., EBFP, ECFP, EYFP, Cerulaen, mTurquoise2, YFP, EYFP, mCitrine, Venus, YPet, Superfolder GFP, mCherry, - 5 - mPlum) are meant by the term "fluorescent dye" in the sense of this document. Further from the group of fluorescent proteins the term "fluorescent dye" in the sense of this document may include fluorescent proteins, whose absorbance or emission characteristics change upon binding of ligand such as BFPms1 or in response to changes in the environment such as redox-sensitive roGFP or pH sensitive variants. Further from the group of fluorescent proteins the term "fluorescent dye" in the sense of this document may include derivatives of cyanobacterial phycobiliprotein small ultra red fluorescent protein smURFP as well as fluorescent protein nanoparticles that can be derived from smURFP. An overview of fluorescent proteins can be found in the article of Rodriguez et al. in Trends Biochem Sci. 2017 Feb; 42(2): 111-129. A fluorescent dye in the sense of this document may further refer to a fluorescent quantum dot. A fluorescent dye in the sense of this document may further refer to fluorescent carbon dot, a fluorescent graphene quantum dot, a fluorescent carbon-based nanostructure as described in the article of Yan et al. in Microchimica Acta (2019) 186: 583 and Iravani and Varma 2020 in Environ Chem Lett. 2020 Mar 10 : 1-25. A fluorescent dye in the sense of this document may further refer to a fluorescent polymer dot (Pdot) or nanodiamond. A fluorescent dye in the sense of this document may further refer to a fluorescent dyad, such as a dyad of a perylene antenna and a triangelium emitter as described in the article of Kacenauskaite et al. in J. Am. Chem. Soc. 2021, 143, 1377-1385. A fluorescent dye in the sense of this document may further refer to an organic dye, a dyad, a quantum dot, a polymer dot, a graphene dot, a carbon-based nanostructure, a DNA origami-based nanostructure, a nanoruler, a polymer bead with incorporated dyes, a fluorescent protein, an inorganic fluorescent dye, a SMILE, or a microcapsule filled with any of the aforementioned. A fluorescent dye in the sense of this document may further refer to a FRET-pair having at least one fluorescent dye as FRET donor and at least one fluorescent dye as a FRET acceptor, or a FRET-triple, which is used to generate a three component Förster resonance energy transfer. In particular, the FRET-pair or FRET-triplet is connected by a complementary linker or by a linking element. A fluorescent dye in the sense of this document may further refer to a FRET n-tupel of physically connected dyes. A fluorescent dye may also be a polymer dye.

**Förster Resonance Energy Transfer (FRET)** has been widely used to measure protein-protein interactions using both fluorescent proteins and fluorescent dyes. The spatial stringency of FRET is in the range of 10nm. In other words, the FRET efficiency E E= 1/(1+(r/R0)^6 ) drops exponentially with an increase in donor and acceptor dye distance r, such that effective FRET typically occurs when the donor and acceptor dye are within 1 to 10nm distance r to each other, wherein R0 is the Förster Distance of the donor-acceptor dye pair. Typically, the donor and acceptor dye or fluorophore are different fluorophores. For example the following dye pairs are commonly used FRET pairs: ATTO 425 - ATTO 520, ATTO 488 - ATTO 550, ATTO 488 - ATTO 565, ATTO 488 - ATTO 647N, ATTO 488 - ATTO 655, ATTO 520 - ATTO 647N, ATTO 532 - ATTO 647N, ATTO 532 - ATTO 655, ATTO 550 - ATTO 590, ATTO 550 - ATTO 647N, ATTO 565 - ATTO 590, ATTO 565 - ATTO 647N, ATTO 590 - ATTO 620, ATTO 590 - ATTO 647N, ATTO 590 - ATTO 680, ATTO 620 - ATTO 680, Alexa Fluor 488 - Alexa Fluor 555, Alexa Fluor 594 - Alexa Fluor 647, Fluorescein - Tetramethylrhodamine. Likewise, commonly used fluorescent protein FRET pairs exists including but not limited to: ECFP-EYFP, mTurquoise2-mVenus, EGFP-mCherry, mNeonGreen-mRuby3. Quantum dots may also be used as donor or acceptors. The use of quantum dots as FRET donors is particularly advantageous because of the high extinction coefficients, which leads to brighter FRET signals. Various methods exist to measure the FRET efficiency including but not limited to measuring the intensity of the acceptor emission (sensitized emission), photobleaching FRET wherein the donor is bleached and bleaching rates differ depending on the presence or absence of the acceptor, fluorescence lifetime measurements that register the change in the lifetime of the donor (i.e. when FRET occurs the donor lifetime shortens; FRET-FLIM). Likewise, FRET may be assessed by measuring donor dequenching following the removal or bleaching of the acceptor (e.g. acceptor photobleaching). In addition to these methods FRET may also be measured by anisotropy imaging like for example in the case of Homo-FRET, i.e. in assays wherein the donor and acceptor are of the same fluorophore. While these aforementioned methods generally readout pluralities of donor and acceptor dye molecules, methods to measure single molecule FRET have likewise been developed. A simple method to measure FRET is ratiometric imaging of donor and acceptor intensity (sensitized emission), which can be performed on simple channel-based readouts like widefield microscopes, cytometers or plate readers. While measuring FRET efficiency or FRET signal in this way by measuring the donor and acceptor emission is simple, it is connected to the challenge of donor crosstalk into the acceptor emission channel, which may also be named the FRET channel. This is why more sophisticated methods like measurement of the donor dequenching were developed. Alternatively, spectral imaging offers a good solution to the crosstalk problem in FRET measurements, but suitable instrumentation may not always be available. In tissue sections, however, it is difficult to perform FRET measurements, because of the high background autofluorescence. While FRET has been performed in tissue sections before, it is known in the field and also evident from the published data that FRET-based assays in tissue sections are limited by poor signal-to-noise or signal-to-background, because of the high background autofluorescence that is commonly found in tissue samples. Reference is made to König P, Krasteva G, Tag C, König IR, Arens C, Kummer W., FRET-CLSM and double-labelling indirect immunofluorescence to detect close association of proteins in tissue sections, Lab Invest. 2006 Aug;86(8):853-64. doi: 10.1038/labinvest.3700443. Epub 2006 Jun 19. PMID: 16783395.

### Example PHA protocols

The following compositions are provided for the sake of providing an example. The person skilled in the art will easily find new additives and make changes to salt concentrations, blocking agent concentrations, chaotropic agents and the like to adapt these compositions to the needs of the particular application or biological sample without deviating from the invention.

### Example Blocking buffer:

### Base component:

5 % normal goat serum/PBS or
5 % normal donkey serum/PBS or
1 % BSA/PBS
137mM - 500mM NaCl
0.2-10mg/ml Salmon sperm DNA (e.g. from THERMO #AM9680)
1-10nmol/ml Scrambled oligonucleotide ssDNA (e.g. from IDT, Iowa)
Optional further additives:
DMSO
Detergents (Triton-X100, NP-40, saponin, Tween-20)

### Options for degradation agent - blocking strand configurations:

(1) First and second label part are DNase-I resistant. In this case the blocking strand can be removed by application of DNase I.
(2) First and second label part comprise DNA, blocking strand(s) comprise RNA. In this case the blocking strands can be removed with RNase H.
(3) Blocking strand(s) comprise at least one disulfide bond in its backbone. In this case the blocking strands can be cleaved with TCEP.
(4) Blocking strand(s) comprise at least one UV-cleavage site in its backbone. In this case the blocking strands can be cleaved with UV-light.

### Example 1: Proximity Hybridization Assay (PHA) for direct detection of analyte proximity in cells:

### Preparation:

(A) Prepare oligonucleotide barcoded primary antibody pairs. These may be either procured directly from various vendors or can be generated using available antibody oligonucleotide conjugation kits (e.g. Oligonucleotide Conjugation Kit **ab218260** from abcam (Cambridge, UK)). Oligonucleotides with suitable functional groups may be procured from IDT (Coralville, USA) or Biomers (Ulm, Germany). Likewise, oligonucleotide barcoded primary antibodies may be obtained by using site-specific conjugation.
(B) Prepare first and second label parts comprising complementary barcodes to allow later conjugation to antibody pairs mentioned in (A) via hybridization. This is a particularly efficient way of working with the method. Nevertheless, it is also possible to conjugate the first and second label parts directly and covalently using the aforementioned kit or other chemistries like click chemistry and suitably bi-functional linkers (e.g. NHS-PEG(n)-N3 linkers).
(C) Depending on the expression level(s) of the target(s) it may be desirable to amplify the signal. This may be achieved in at least two different ways: (1) by using dendrimeric oligonucleotide-based or other DNA-scaffold structures e.g. nanorulers, DNA origami-, DNA-brick-based structures, (2) by amplifying the barcode sequences using an enzymatic method like rolling circle amplification (RCA), or loop-mediated isothermal amplification (LAMP), or polymerase chain reaction (PCR) for example.

### Main protocol:

1. Wash the cells twice.
2. Fix with 4 % formaldehyde for 10 minutes and wash 3 ×.
3. Permeabilize with 0.1 % TX-100/PBS for 15-20 minutes and wash 3 ×.
4. Block with for 45 minutes in blocking buffer.
5. Dilute the oligonucleotide barcode-conjugated primary antibodies in **Blocking buffer** and apply it for 1-2 h (or overnight at 4 °C). Typically, dilutions are in the range of 1:100-1:500.
6. Wash 4 × thoroughly to remove unbound primary antibody with PBS or **Blocking buffer** each with agitation.
7. Dilute first and second label parts comprising complementary barcode sequences (i.e. complementary to the barcodes on the primary antibodies) in **Blocking buffer.**
8. Apply first and second label parts diluted in **Blocking buffer** to the sample and allow to hybridize to the respective barcodes on the target-bound primary antibodies. This step may be carried out at room temperature for 5-60min or at a higher temperature.
9. Wash 4x with **Blocking buffer or a wash buffer of choice or PBS** to remove unbound first and second label parts each with agitation. During this step one may also include DAPI for nuclear staining.
10. Optionally, perform a first readout and/or image the sample before applying the degradation agent and/or removing the blocking strand(s). This readout/image may be used as a baseline and may improve the quality of the assay, but is not required in many cases.
11. Apply the suitable condition to remove and/or degrade the blocking strands.
12. Optionally, wash the sample with a blocking buffer, wash buffer of choice, or PBS.
13. Allow the hybridization of the first and second label parts to occur, which may typically require 5-60min.
14. Optionally, provide a drop of mounting medium on a microscope slide and lay the coverslip with the cells or the biological sample upside down on this drop. Press the specimen with the tweezers slightly so that the mounting medium is well distributed, without squeezing the sample. The preparation is ready for microscopy after curing.
15. For optical-grade carrier bottom plates, dishes, or flow cells samples may be imaged directly in the **blocking buffer, wash buffer,** or in PBS, or any other imaging buffer of choice, so as long as the imaging buffer does not contain anything that disturbs the formed duplex between the first and second label parts.
16. Perform a readout and/or image the sample.
17. Optionally, perform a comparison of the pixel intensities before and after allowing the duplex formation between the first and second label parts, i.e. under the complexing condition and decomplexing condition to calculate a fold change or ratio of the (FRET) signal on a pixel-per-pixel basis to assess the degree of proximity of the markers and thereby of the analytes.

### Short Description of the Figures

Hereinafter, specific embodiments are described referring to the drawings, wherein:
- Figure 1: is a schematic view of a marker comprising a label for analysing a biological sample,
- Figure 2: is a schematic view of the marker according to Fig. 1,
- Figure 3: is a schematic view of the marker according to Fig. 1,
- Figure 4: is a schematic view of the marker according to Fig. 1,
- Figure 5: is a schematic view of a marker according to a second embodiment,
- Figure 6: is a schematic view of a marker according to a third embodiment,
- Figure 7: is a schematic view of different proximity hybridization assay (PHA) formats,
- Figure 8: is a schematic view of a proximity hybridization assay (PHA) with a guest-modified blocking strand,
- Figure 9: is a schematic view showing the use of proximity hybridization assays (PHA) for assessing genetic modifications, and
- Figure 10: is a schematic view showing the use of proximity hybridization assays (PHA) for gene and cell therapy quality control and monitoring.

### Detailed Description

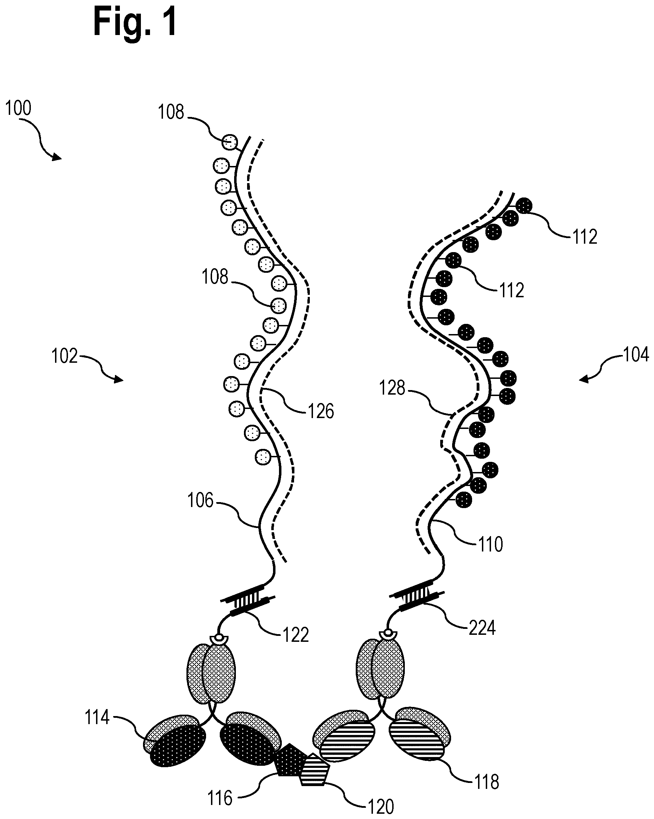

Figure 1 is a schematic view of a marker 100 for analysing a biological sample. The marker 100 comprises a first marker part 102 and a second marker part 104. The first marker part 102 may comprise a first label part with a first nucleic acid strand 106, and a plurality of first labelling moieties 108. The first labelling moieties 108 may be (covalently) attached to the first nucleic acid strand 106.

The second marker part 104 comprises a second label part with a second nucleic acid strand 110, and a plurality of second labelling moieties 112 attached to the second nucleic acid strand 110. The first nucleic acid strand 106 and the second nucleic acid strand 110 may be polynucleotides, for example.

The first nucleic acid strand 106 and the second nucleic acid strand 110 are at least partially complementary to each other. This enables hybridisation of the first nucleic acid strand 106 and the second nucleic acid strand 110, in particular to form a duplex based on Watson-Crick base pairing.

The first marker part 102 further comprises a first affinity reagent 114 configured to specifically bind to a first target analyte 116. Similarly, the second marker part 104 further comprises a second affinity reagent 118 configured to specifically bind to a second target analyte 120.

The first label part is attached to the first affinity reagent 114 via a barcode oligonucleotide 122 of the first affinity reagent 114. Similarly, the second label part is attached to the second affinity reagent 118 via a barcode oligonucleotide 124. Each barcode oligonucleotide 122, 124, in particular its sequence of nucleotides, is preferably specific to the respective affinity reagent 114, 118 and/or the respective label part, in particular to the first and second nucleic acid strand 106, 110, respectively. In particular, the first and second affinity reagent 114,118 are antibodies.

The first marker part 102 is bound to the first target analyte 116 via the first affinity reagent 114. Similarly, the second marker part 104 is bound to the second target analyte 120 via the second affinity reagent 118.

The marker 100 further comprises a first blocking nucleic acid strand 126 and/or a second nucleic acid strand 128. The blocking nucleic acid strands 126, 128 are configured to either hybridise to the first nucleic acid strand 106 or the second nucleic acid strand 110. In Fig. 1 the first blocking nucleic acid strand 126 is shown to be hybridised to the first nucleic acid strand 106 and the second blocking nucleic acid strand 128 is shown to be hybridised to the second nucleic acid strand 110. The presence of the blocking nucleic acid strands 126, 128 and their hybridisation to the respective nucleic acid strand 106, 110 means that the first nucleic acid strand 106 and the second nucleic acid strand 110 are prevented from directly hybridising to each other. Thus, when handling the marker parts 102, 104, for example, when preparing to analyse a biological sample, the marker parts 102, 104 do not hybridise to each other due to the blocking nucleic acid strands 126, 128.

In an alternative embodiment, the marker 100 may only comprise either one of the blocking nucleic acid strands 126,128. This single blocking nucleic acid strand 126,128 may nevertheless achieve the abovementioned effect of hindering the hybridisation of the first nucleic acid strand 106 with the second nucleic acid strand 108.

In the exemplary embodiment of Fig. 1, the target analytes 116, 120 are in contact, for example, due to an affinity interaction between the target analytes 116, 120. This results in the marker parts 102, 104 to be in proximity once they are bound to the target analytes 116, 120. As described above, despite their proximity, the first nucleic acid strand 106 and the second nucleic acid strand 110 do not hybridise to each other due to the blocking nucleic acid strands 126, 128. This enables easy handling of the marker 100, in particular its marker parts 102, 104 during an analysis of a biological sample containing the target analytes 116, 120. For example, in case the marker parts 102, 104 are not yet bound to the target analytes 116, 120 when introducing the marker 100 into a (in Fig. 1 not shown) biological sample, the blocking nucleic acid strands 126, 128 prevent the undesired uncontrolled hybridisation of the first nucleic acid strand 106 and the second nucleic acid strand 110.

The first labelling moieties 108 and the second labelling moieties 112 may be fluorophores that act as FRET donors or FRET acceptors, respectively. Thus, each first labelling moiety 108 may form a FRET pair with one of the second labelling moieties 112. Since the first nucleic acid strand 106 and the second nucleic acid strand 110 are not hybridised in the state shown in Fig. 1, the first nucleic acid strand 106 and the second nucleic acid strand 110 are not in close proximity to each other. In particular, the first labelling moieties 108 and the second labelling moieties 112 are not within a distance that enables a FRET to occur. Such a distance is frequently in the range between 1 and 10 nm. Thus, the first labelling moieties 108 and the second labelling moieties 112 might be optically detectable as (single) ordinary fluorescent dyes but not according to the FRET characteristic.

Additionally, guest molecules (not shown) may be optionally attached to at least one of the blocking nucleic acid strands 126, 128. For example, the guest molecules may be covalently attached to nucleotides of the blocking nucleic acid strands 126, 128. In particular, one guest molecule may be provided for every 5 to 10 nucleotides of the respective blocking nucleic acid strand 126, 128. The marker 100 may further be optionally provided with host molecules (not shown). The guest molecule and the host molecule are configured to form a guest-host complex (not shown). This, this enables generating a host-guest complex, which prevents the hybridisation of the blocking nucleic acid strand 126, 128 to the first nucleic acid strand 106 or the second nucleic acid strand 110, or which causes the dissociation of the blocking nucleic acid strand 126, 128 from the first and/or second nucleic acid strand 106, 110.

The interaction between guest molecules and host molecules is a fundamental concept in supramolecular chemistry, where the host molecules provide a structural framework that encapsulates or binds the guest molecules through non-covalent interactions. These interactions can include hydrogen bonding, van der Waals forces, electrostatic interactions, and hydrophobic effects. The specificity and strength of the interaction depend on the complementarity between the host and guest in terms of shape, size, and functional groups. For example, cyclodextrins may act as host molecules that can form inclusion complexes with a variety of guest molecules, such as aromatic compounds, and fatty acids.

Another example is cucurbit[n]urils, a family of macrocyclic molecules, which can encapsulate guests ranging from small metal ions to large organic molecules like drugs and dyes, forming highly stable complexes. Examples of small organic molecules that form stable complexes with cucurbit[7]urils include adamantane and ferrocene. Examples of aromatic compounds that form stable complexes with cucurbit[7]urils include methyl viologen and 1,4-dimethoxybenzene. Specific examples of suitable guest molecules include 1-adamantanemethylamine, ferrocenyl methylamine, 1,4-benzenedimethanamine, and 4-tertbutylbenzylamine.

In particular, Fig. 1 shows a step of a method for analysing a biological sample (not shown) according to which the marker 100 may be introduced into a biological sample to be analysed. The blocking nucleic acid strands 126, 128 prevent undesired hybridisation of the first and second nucleic acid strands 106, 110 before the marker 100 is introduced into the biological sample and the affinity reagents 114, 118 have bound to their respective target analytes 116, 120.

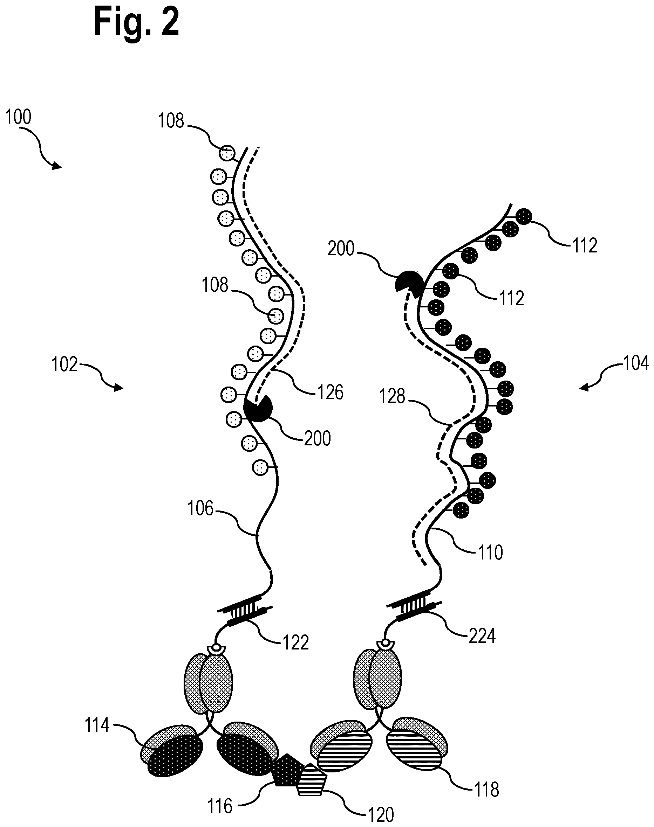

Figure 2 is a schematic view of the marker 100 in the presence of a degradation agent 200. In particular, the degradation agent 200 is a nuclease. The degradation agent 200 can (specifically) degrade or digest the blocking nucleic acid strands 126, 128 and thereby remove it. In particular, the degradation agent 200 may be added to a biological sample to be analysed as part of a method for analysing the biological sample.

In order to enable selective degradation of only the blocking nucleic acid strands 126, 128, the blocking nucleic acid strands 126, 128 may be configured to be sensitive to the degradation agent 200 and the first and second nucleic acid strands 106, 110 may be configured to be resistant to the degradation agent 200. For example, the first and second nucleic acid strands 106, 110 (and the barcode oligonucleotides 122, 224) may consist essentially of analogous nucleic acids such as non-natural nucleic acids, which are resistant to a particular degradation agent 200, such as the nuclease. Specific examples of analogous nucleic acids include L-DNA, and peptide nucleic acids. Conversely, the blocking nucleic acid strands 126, 128 may consist essentially of natural nucleic acids such as DNA, which are sensitive to a particular degradation agent 200, such as the nuclease.

Alternatively, the degradation agent may be a physical stimulus such as a UV light impulse and the blocking nucleic acid strands 126, 128 comprise nucleotide analogues that are sensitive to UV light.

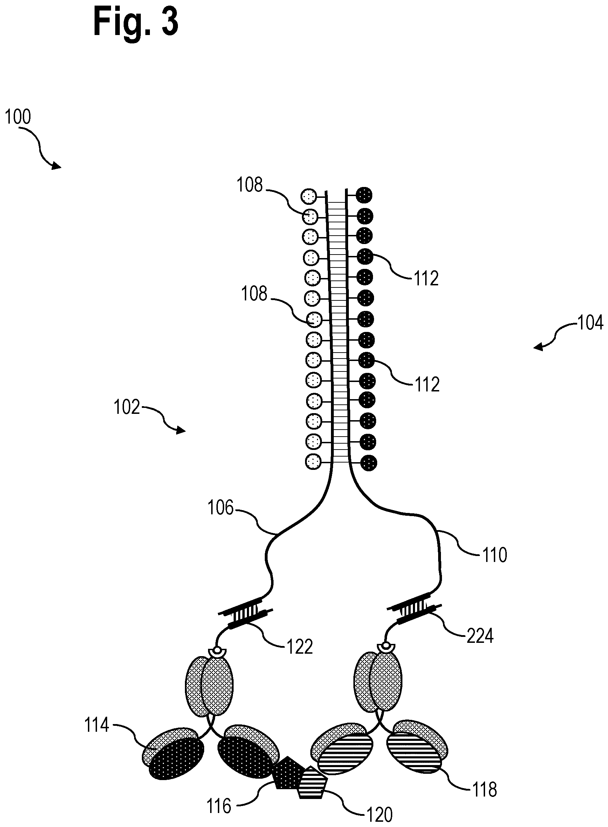

Figure 3 is a schematic view of the marker 100 with the blocking nucleic acid strands 126, 128 removed, for example, due to the previous addition of the degradation agent 200 as described for Fig. 2. Without the blocking nucleic acid strands 126, 128 present, the first and second nucleic acid strands 106, 110 may freely hybridise to each other. In Fig. 3 the first and second nucleic acid strands 106, 110 are shown to be hybridised to each other.

The hybridisation of the first nucleic acid strand 106 and the second nucleic acid strands 110 brings the first labelling moieties 108 and the second labelling moieties 112 into proximity such that a FRET may occur between the first labelling moieties 108 and the second labelling moieties 112. Thus, the proximity of the target analytes 116, 120 may be determined by the occurrence of FRET between the first labelling moieties 108 and the second labelling moieties 112, for example, by means of an optical readout with a fluorescence microscope. In Fig. 3, a FRET can occur between all the first labelling moieties 108 and the second labelling moieties 112.

In a method for analysing a biological sample, the marker 100, in particular the marker parts 102, 104, may initially be introduced into the biological sample in the presence of the blocking nucleic acid strands 126, 128, as shown in Fig. 1. After waiting for an appropriate amount of time for the marker parts 102, 104 to bind, any marker parts 102, 104 that remain unbound to a respective target analyte 116, 118 may be removed, for example by washing the biological sample.

Subsequently, the blocking nucleic acid strands 126, 128 may be degraded as described for Fig. 2, which results in their removal. Fig. 3 shows the marker 100 after removal of the blocking nucleic acid strands 126, 128.

This in turn enables hybridisation of the first nucleic acid strand 106 with the second nucleic acid strands 110. Subsequently, an optical readout may be generated of the biological sample with the marker 100. The optical readout may be an image, for example, generated by means of a fluorescence microscope.

The optical readout enables determining whether or not the target analytes 116, 120 are in close proximity to each other. In particular, the optical readout can be used to determine whether or not a FRET occurs between the first labelling moieties 108 and the second labelling moieties 112, by observing a respective change in their optical properties due to the FRET.

In a different embodiment, the marker 100, in particular the marker parts 102, 104, may initially be introduced into the biological sample with the marker 100 comprising guest molecules. During the step of removing the blocking nucleic acid strands 126, 128 the host molecules may be added, and the guest-host complex may form, in particular in order to reduce a melting temperature of the hybridised blocking nucleic acid strands 126, 128. The reduction in the melting temperature enables the easier removal of the blocking nucleic acids strands 126, 128 from the first and second nucleic acid strand 106, 110.

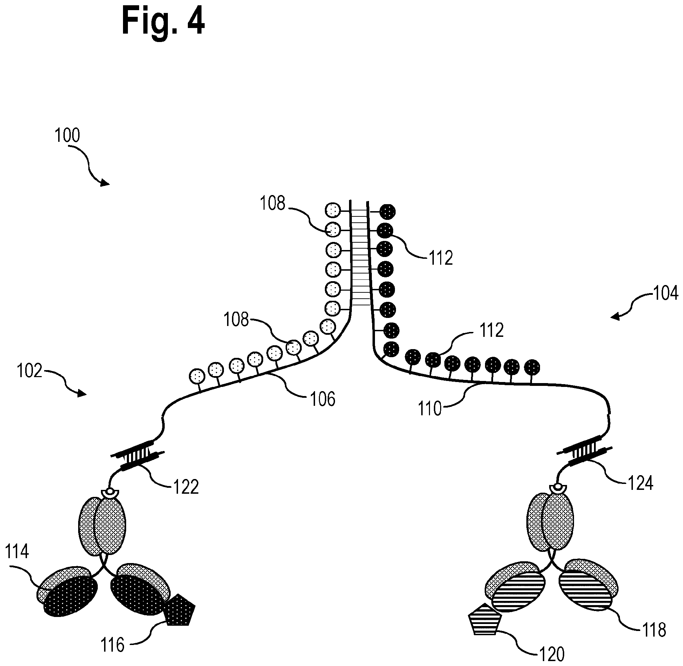

Figure 4 is a schematic view of the marker 100 with the target analytes 116, 120 at a greater distance from each other compared to the Figs. 1 to 3. Similarly to Fig. 3, the blocking nucleic acid strands 126, 128 are not present and the first nucleic acid strand 106 and the second nucleic acid strand 110 can hybridise to each other. However, the greater distance between the target analytes 116, 120 results in the first nucleic acid strand 106 and the second nucleic acid strand 110 to only partially hybridise, in particular to hybridise to a lesser extent compared to the state shown in Fig. 3, in which the target analytes 116, 120 are in contact with each other.

The partially hybridised first nucleic acid strand 106 and second nucleic acid strand 110 results in some of the first labelling moieties 108 and the second labelling moieties 112 to be at a greater distance from each other. In particular, these first labelling moieties 108 and second labelling moieties 112 may at a distance from each other, at which no FRET occurs between them. Thus, the overall observable FRET efficiency is reduced compared to the state shown in Fig. 3. This reduced FRET efficiency can be observed when generating an optical readout of the biological sample with the marker 100. Thus, the first labelling moieties 108 and the second labelling moieties 112 which do not form a FRETR pair - e.g. the labelling moieties 108, 112 between the barcode oligonucleotides 122, 124 and the hybridized part of the first nucleic acid strand 106 and the second nucleic acid strand 110 - might be optically detectable as (single) ordinary fluorescent dyes according to their (spectral) emission characteristic of the fluorescent dyes, whereas the first labelling moieties 108 and the second labelling moieties 112 which do form a FRETR pair - e.g. the labelling moieties 108, 112 of the hybridized part of the first nucleic acid strand 106 and the second nucleic acid strand 110 - can be detected according to the FRET characteristic of the FRET pair and therefore according to a different spectral characteristic of the FRET pair compared to the spectral characteristic of the single fluorescent dyes. The ratio between the two different spectral characteristics can be used as a quantitive measure of the respective proximity of the affinity reagents 114, 118 and therefore of the target analytes 116, 120.

In summary and with particular reference to Figs. 3 and 4, it can be seen that the distance between the target analytes 116, 120 has a direct effect on the degree of hybridisation between the first nucleic acid strand 106 and second nucleic acid strand 110 and therefore on the overall FRET efficiency between the first labelling moieties 108 and second labelling moieties 112. The FRET efficiency between the first labelling moieties 108 and second labelling moieties 112 is therefore proportional to the distance between the target analytes 116, 120 and can be used to determine the distance or proximity between the target analytes 116, 120.

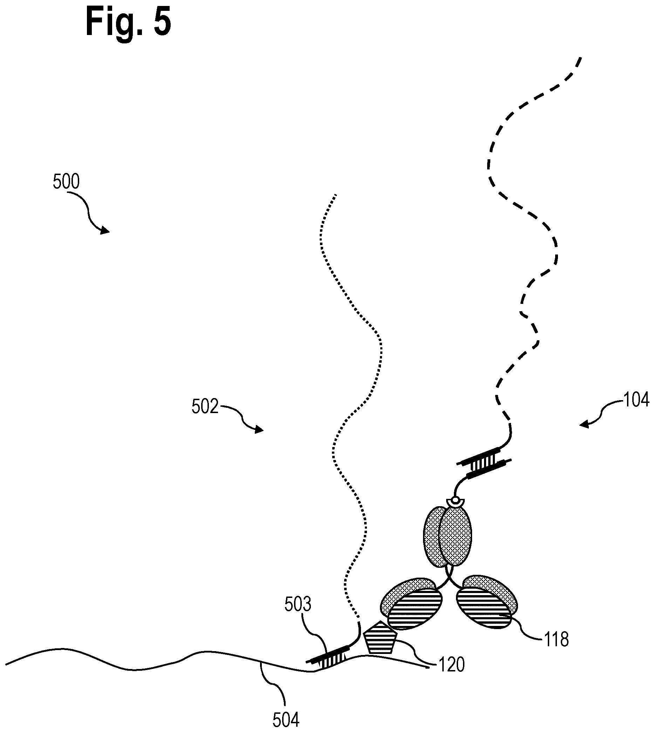

Figure 5 is a schematic view of a marker 500. The marker 500 comprises a first marker part 502 with an affinity reagent 503 that is a linear nucleic acid. The marker 500 further comprises the second marker part 104 described above. For simplicity no labelling moieties are shown in Fig. 5 for the marker 500.

The first marker part 502 binds specifically to a nucleic acid target analyte 504. The second marker part 104 binds specifically to the second target analyte 120, which may be a protein. The target analytes 504, 120 may interact with each other or bind to each other. This interaction or proximity between the target analytes 504, 120 may be determined by the marker 500 in the same manner as described for the marker 100. In particular, the proximity of respective labelling moieties (not shown) of the marker parts 502, 104 may be determined by FRET occurring between the labelling moieties as a measure of the distance between the target analytes 504, 120.

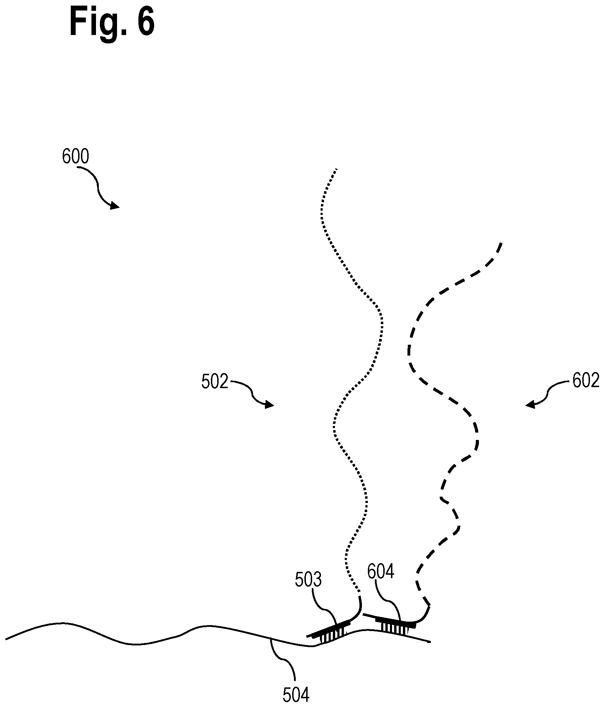

Figure 6 is a schematic view of a marker 600. The marker 600 comprises the first marker part 502 and a second marker part 602 with first and second affinity reagents 503, 604 that are linear nucleic acids, respectively. For simplicity no labelling moieties are shown in Fig. 6 for the marker 600.

Both affinity reagents 503, 604 are configured to specifically bind to a particular target sequence. The nucleic acid target analyte 504 comprises these target sequences. By means of the marker 600 the proximity between the target sequences along the target analyte 504 may be determined or the presence of the target analyte 504 may be determined with high confidence due to the use of two affinity reagents that need to bind to the target analyte 504 simultaneously. The marker 600 may be detected as described for the markers 100, 600 above. In particular, the proximity of respective labelling moieties (not shown) of the marker parts 502, 602 may be determined by FRET occurring between respective labelling moieties (not shown) of the marker parts 502, 602 as a measure of the distance between the target sequence or as a measure of the presence of the target analyte 504 in the biological sample.

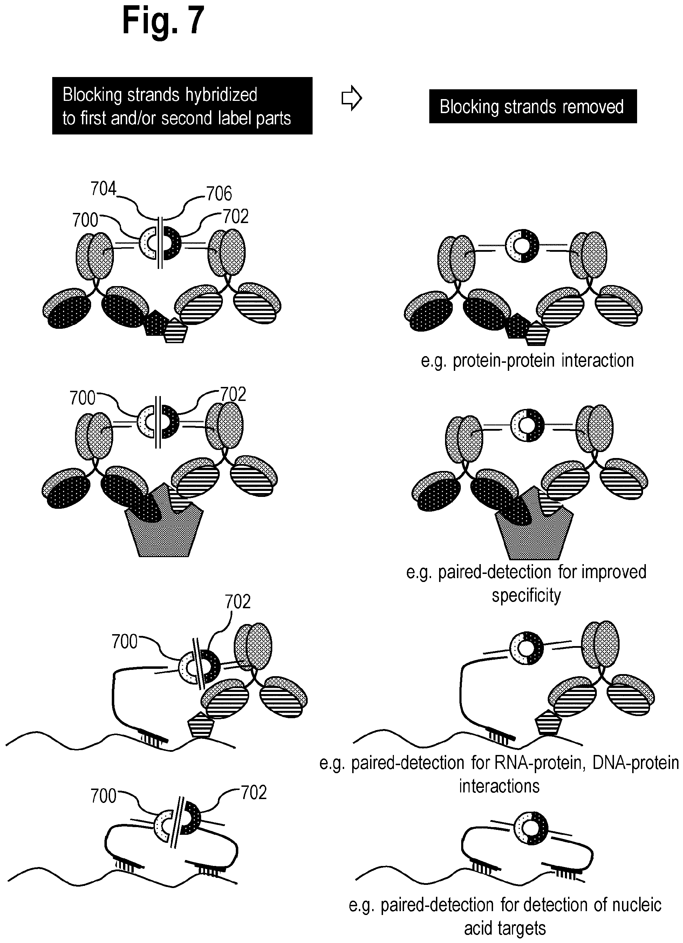

Figure 7 is a schematic view of different embodiments of markers for proximity hybridisation assays. Fig. 7 therefore also shows different embodiments of proximity hybridization assays (PHA). In Figure 7 the first and second label parts are schematically shown as half donut-shapes and respectively indicated in Fig. 7 by reference signs 700, 702. The left column of Fig. 7 shows a markers in a first condition, wherein at least one of the first and second label parts 700, 702 of each marker, in particular their respective first and second nucleic acid strands, is hybridized to a blocking nucleic acid strand 704, 706 or a plurality of blocking nucleic acid strands. For simplicity these may be referred to simply as blocking strands and the first condition may be referred to as the blocked condition or blocked label part(s). The right column of Fig. 7 shows each marker under a second condition or unblocked condition or unblocked label part(s) after the removal of the blocking strands.

The embodiment in the first row of Fig. 7 shows a PHA assay used for protein-protein proximity and may be used as a proxy for protein-protein interactions. The second row shows a PHA assay for paired detection of an analyte like a protein with two different affinity reagents binding to distinct sites or epitopes of the target protein. This may be used to enhance specificity or to interrogate posttranslational modifications e.g. phosphorylation, ubiquitination, sumoylation, acetylation, methylation, proteolytic processing or alternative splicing. The embodiment shown in the third row shows a PHA assay for protein-RNA or protein-nucleic acid interaction and may be used to assess for example promoter occupancy of biomarker genes with certain transcription factors. The embodiment shown in the fourth row is a PHA assay that allows the detection of proximities between two distinct nucleic acid target sequences.

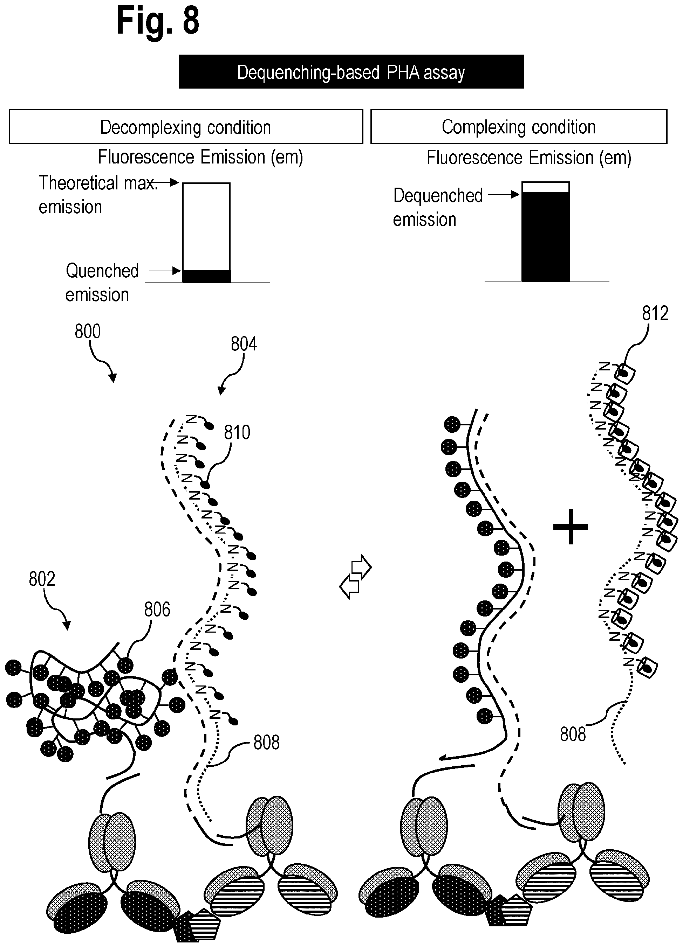

Figure 8 is a schematic view of a marker 800 that is used for a dequenching-based PHA assay. The marker 800 comprises the first marker part 802 and a second marker part 804 with respective first and second affinity reagent. The first and second marker parts 802, 804 comprise respective first and second label parts. In this example only the first label part of the first marker part 802 comprises the first and second labelling moieties 806, which are the same dye, in particular of the same type. For example, this may be a hydrophobic fluorescent dye like ATTO647N. Due to the hydrophobic nature of the ATTO467N dye molecules, the first label part is a quenched label. In other words, it can be excited, and its emission can be detected, but the emission intensity is quenched and lower than the dequenched emission or the theoretical maximum. The fold change between quenched and dequenched emission depends on the number of dye molecules attached to the first label part, the dye-to-dye spacing, and the difference of the persistence length of the first nucleic acid strand in the single stranded and duplex condition. In the example shown in Figure 8, only the second label part is hybridized to a blocking nucleic acid strand(s) 808, which comprises at least one guest-modified nucleotide 810 configured to complexate with a host molecule 812, e.g. cucurbit[7]uril (CB[7]). This configuration allows the removal of the blocking strand(s) 808 by an increase in the host molecule 812 concentration (complexing condition, shown on the right column). As shown by Xiao et al. 2022, the guest-modified nucleotides (e.g. FC, AD) and host molecules (e.g. CB[7]) can be used to control the hybridization of nucleic acid strands. The embodiment of the label, marker 800, and PHA assay shown in Figure 8 exploits this to generate blocking strand(s) 808 that can be removed by addition of CB[7] or other suitable host molecules 812. This is advantageous, as it allows the gentle and rapid removal of the blocking strands 808.

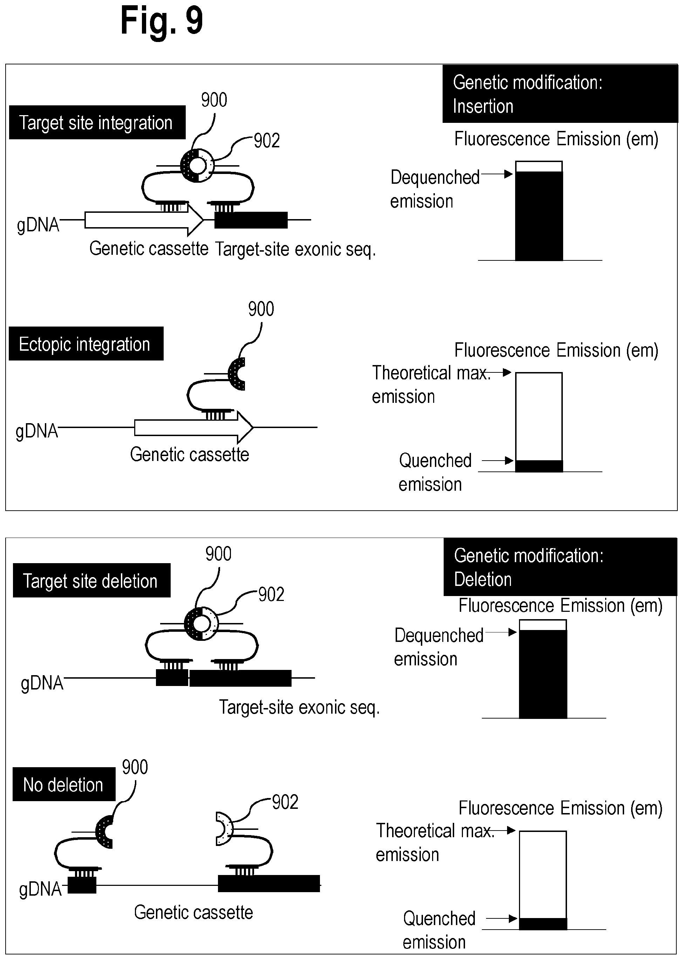

Figure 9 is a schematic view of a PHA assay that is used for assessing the integration of genetic cassettes that are used in gene and cell therapy. These assays are based on using oligonucleotides as affinity reagents and single molecule fluorescent in situ hybridization (smFISH).

The top row of the top panel of Fig. 9 shows the target site integration of the genetic cassette (white arrow) next to an exon (black rectangle) the genomic DNA is otherwise depicted as a black line. The bottom row of the top panel of Fig. 9 shows a random or ectopic integration. PHA assays allow the faithful separation of the distinct classes of integrations as only the target site integrations are detected with both label parts 900, 902 of the marker, whereas in a random integration only the first or second label part 900 ,902 is present. Following to the removal of the blocking strands the target site integration is detectable as a fluorescent spot, which is brighter than the one of an ectopic integration when dequenching-based PHA assays are used and/or which has a higher FRET efficiency or different fluorescent lifetime, when FRET-based PHA assays are used.

The bottom panel of Fig. 9 shows a schematic view of a PHA assay that is used for assessing the deletion of genomic sequences. A part of the genome may for example be deleted in a cell line to correct a genetic defect. This can be achieved by for example using the CRISPR/Cas9 or similar systems as well as by leveraging homologous recombination. If patient-derived cells for example are modified to delete a certain part of the genome it may be desirable to assess the frequency which with the appropriate deletion occurs. This may be achieved by using the PHA assay schematically shown in the bottom panel. Due to the deletion of the target site both probes of the first and second marker part are brought into proximity, which can be readout out as a corresponding smFISH spot, will be brighter than the one of a site that has no deletion when dequenching-based PHA assays are used and/or which has a higher FRET efficiency or different fluorescent lifetime, when FRET-based PHA assays are used.

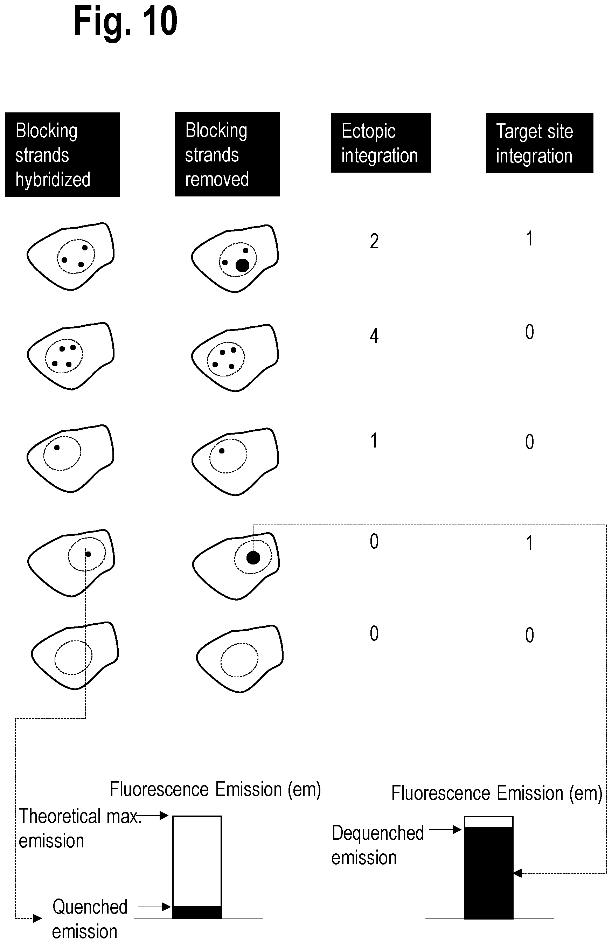

Figure 10 is a schematic view of a hypothetical readout of the assay described in Figure 9. A plurality of cells is analyzed, which may for example be derived from a blood sample to monitor the evolution of a cell therapy product in a patient. Such a monitoring may be performed to assess how distinct clones in a cell therapy product evolve in a patient during the course of a cell therapy. Such clones may comprise no genetic modification (e.g. no insertion/integration or deletion), they may comprise only the desired target-site modification, they may comprise only ectopic integrations and/or deletions and they may comprise a mix of target-site and ectopic modification. Cells are stained and readout before and after removal of the blocking strands, spots are segmented and the before and after images are registered to identify the spots that change following to the removal of the blocking strands indicating a paired detection event.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 500, 600, 800: Marker
- 102, 502, 700, 802, 900: First marker part
- 104, 602, 702, 804, 902: Second marker part
- 106: First nucleic acid strand
- 108, 806: First labelling moiety
- 110: Second nucleic acid strand
- 112, 806: Second labelling moiety
- 114, 503: First affinity reagent
- 116, 504: First target analyte
- 118, 604: Second affinity reagent
- 120: Second target analyte
- 122, 124, 224: Barcode oligonucleotide
- 126, 704: First blocking nucleic acid strand
- 128, 706: Second blocking nucleic acid strand
- 200: Degradation agent
- 808: Guest-modified blocking nucleic acid strand
- 810: Guest molecule
- 812: Host molecule

## Claims

1. A label for analysing a biological sample comprising:
a first label part (102) comprising a first nucleic acid strand (106), and
a second label part (104) comprising a second nucleic acid strand (110), and
wherein the first nucleic acid strand (106) and the second nucleic acid strand (110) are configured to form a duplex,
wherein the label further comprises at least one first labelling moiety (108) and at least one second labelling moiety (108, 112), and
wherein the label further comprises at least one blocking nucleic acid strand (126, 128, 704, 706, 808) at least partially complementary to one of the first nucleic acid strand (106) and the second nucleic acid strand (110).

2. The label according to claim 1, wherein the at least one blocking nucleic acid strand (126, 128, 704, 706, 808) is degradable by means of a degradation agent (200) and wherein the first nucleic acid strand (106) and the second nucleic acid strand (110) are resistant to the degradation agent (200).

3. The label according to one of the preceding claims, wherein the at least one blocking nucleic acid strand (126, 128, 704, 706, 808) consists essentially of one of a nucleic acid analogue or a natural nucleic acid, and the first nucleic acid strand (106) and the second nucleic acid strand (110) consist essentially of the other one of a nucleic acid analogue or a natural nucleic acid.

4. The label according to one of the preceding claims, wherein the at least one blocking nucleic acid strand (126, 128, 704, 706, 808) is complementary to the part of the first nucleic acid strand (106) or the part of the second nucleic acid strand (110) that are complementary to each other.

5. The label according to one of the preceding claims comprising a plurality of blocking nucleic acid strands (126, 128, 704, 706, 808) that each bind to a different part of the first nucleic acid strand (106) or to a different part of the second nucleic acid strand (110).

6. The label according to one of the preceding claims, wherein the at least one first labelling moiety (108) and/or the at least one second labelling moiety (112) is optically detectable.

7. The label according to one of the preceding claims, wherein the at least one first labelling moiety (108) and/or the at least one second labelling moiety (112) preferably comprise identical fluorescent dyes,
or wherein the at least one first labelling moiety (108) comprise at least one first fluorescent dye and the at least one second labelling moiety (112) comprise at least one second fluorescent dye, the at least first and second fluorescent dye having different characteristics.

8. The label according to one of the preceding claims, wherein the at least one first labelling moiety (108) and the at least one second labelling moiety (112) are configured for non-radiative energy transfer between them.

9. The label according to one of the preceding claims, wherein the at least one first labelling moiety (108) and the at least one second labelling moiety (112) are - preferably covalently - both attached to either the first nucleic acid strand (106) or the second nucleic acid strand (110).

10. The label according to one of the preceding claims 1 to 8, wherein the at least one first labelling moiety (108) is - preferably covalently - attached to the first nucleic acid strand (110).

11. The label according to one of the preceding claims, wherein the first nucleic acid strand (106) extends along a first direction and a plurality of the first labelling moieties (108) are arranged on the first nucleic acid strand (106) along the first direction, and/or the second nucleic acid strand (110) extends along a second direction and a plurality of the second labelling moieties (112) are arranged on the second nucleic acid strand (110) along the second direction.

12. The label according to one of the preceding claims, wherein each labelling moiety (108, 112) is equally spaced from any adjacent labelling moiety (108, 112).

13. The label according to one of the preceding claims further comprising at least one guest molecule (810) configured to form a complex with a host molecule (812).

14. The label according to one of the preceding claims 13, wherein the guest molecule (810) is one of 1-adamantanemethylamine, ferrocenyl methylamine, 1,4-benzenedimethanamine, and 4-tertbutylbenzylamine.

15. A marker (100, 500, 600) for analysing a biological sample with a plurality of target analytes (116, 118, 504, 604) comprising:
a label according to one of the preceding claims comprising a first label part and a second label part,
a first marker part (102, 502) comprising a first affinity reagent (114, 503) and the first label part, and
a second marker part (104, 602) comprising a second affinity reagent (118, 604) and the second label part, and
wherein the first affinity reagent (114, 503) and the second affinity reagent (118, 604) are each configured to bind specifically to one of the target analytes (116, 118, 504, 604) of the biological sample.

16. A method for analysing a biological sample, the method comprising the following steps:
introducing at least one marker (100, 500, 600) according to claim 15 into the biological sample,
optionally generating a first optical readout of the biological sample with the marker (100, 500, 600),
removing the blocking nucleic acid strand (126, 128) from the marker (100, 500, 600), and
generating an optical readout or a further optical readout of the biological sample with the marker (100, 500, 600), and
optionally calculating a fold change between the first and second optical readout or comparing the first and second optical readout.

17. A kit for analysing a biological sample comprising a marker (100, 500, 600) according to claim 15 and a degradation agent (200) configured to selectively degrade the blocking nucleic acid strand (126, 128) of the marker.

18. A use of a label according to one of the claims 1 to 14 or a use of a marker according to claim 15 or a use of a kit according to claim 17 for a proximity assay for analysing a biological sample.
